# EUROPEAN PATENT APPLICATION

(11) **EP 2 338 842 A2**
(43) Date of publication of application: **29.06.2011**
(21) Application number: 10006605.9
(22) Date of filing: 24.06.2010
(51) Int. Cl.: C02F 1/467, C02F 1/461, B01D 53/00, A61L 11/00, F26B 9/00, F26B 25/00, C02F 1/28, C02F 103/18, C02F 103/32, C02F 1/00

(54) **Deodorization module**

(30) Priority: 28.12.2009 KR 20090132057; 27.01.2010 KR 20100007550
(71) Applicant: Woongjin Coway Co., Ltd., Gongju-si Chungcheongnam-do 314-895 (KR)
(72) Inventor: Sim, Sang Gu, Seoul, 151-919 (KR); Kim, Sung Jin, Seoul, 151-919 (KR); Park, Chan Jung, Seoul, 151-919 (KR)
(74) Representative: Kador & Partner

(57) **Abstract**

The present invention provides a deodorization module. The deodorization module includes a heat exchanger and an electrolytic cell. The heat exchanger conducts heat exchange between exhaust gas discharged from a drying furnace and cooling air drawn into the heat exchanger from the outside of the drying furnace. The electrolytic cell electrolyzes condensate water, formed by the heat exchange process of the heat exchanger, using bipolar packed bed electrolysis to remove offensive odors from the condensate water. The heat exchanger has flow channels therein so that the exhaust gas discharged from the drying furnace and the cooling air drawn from the outside flows along the flow channels in directions that cross each other.

## Description

### BACKGROUND OF THE INVENTION

### 1. Field of the Invention

The present invention relates generally to deodorization modules and, more particularly, to a deodorization module which can effectively remove offensive odors from high temperature and humidity exhaust gas, generated during a food waste treatment process in a drying furnace, in such a way as to cool the exhaust gas by heat exchange and electrolyze condensate water formed by the cooling.

### 2. Description of the Related Art

Generally, food waste treatment apparatuses discharge high temperature and humidity odorous exhaust gas during the food waste treatment process. It is very difficult to remove offensive odors from exhaust gas because water and gas are discharged quickly. Particularly, as free-standing type food waste treatment apparatuses are recently gaining in popularity, there is an increased interest in treating a large amount of high temperature and humidity gas. Presently, a method of treating exhaust gas using activated carbon and impregnated activated carbon and a method of deodorizing exhaust gas with a high temperature catalytic reaction using platinum are mainly being used. However, these conventional techniques cannot fulfill the requirement that a large amount of high temperature and humidity exhaust gas generated from food waste be able to be treated in a short amount of time. Typically, a large amount of high temperature vapor is generated when food waste is treated. For example, if the dryness factor of a food waste treatment apparatus is 80%, there is about 800 cc of high temperature vapor generated from per kilogram of food waste. Furthermore, high temperature exhaust gas of about 60°C to 80°C is treated by a filter, although the temperature of the exhaust gas may vary depending on the method of pulverizing and drying. A large amount of odorous gas is discharged in mere one to five hours. Due to such operating conditions of the food waste treatment apparatus, the method of deodorizing exhaust gas using activated carbon cannot satisfactorily treat a large quantity of gas. Offensive odors may still be present in the exhaust gas even after it passes through the filter. In addition, although the initial performance of the conventional food waste treatment apparatus may be satisfactory, there is a problem in that the lifetime thereof is short. Moreover, when the activated carbon is impregnated with water, pores of the activated carbon are clogged, resulting in the deodorization performance being deteriorated. Furthermore, because of a separation phenomenon wherein gas which has been held by the activated carbon is re-separated from the activated carbon when the temperature is over 70°C, there is a limitation of the use. In addition, the filter is required to be replaced with a new one once every two to four months, thus inconveniencing a user. A filter using a high temperature catalyst increases the temperature of gas discharged at a temperature over 200°C and removes odorous gas using a platinum catalyst reaction. In this filter, power consumption is comparatively high. Because the temperature of air passing through the filter is very high, even though it is diluted, high temperature air is discharged. Furthermore, the filter increases the temperature of the surroundings of the food waste apparatus and the catalyst reaction generates fine dust. Moreover, the filter must be replaced with a new one at least once per year. In addition, the production cost of the filter using a high temperature catalyst is three to ten times more expensive than the activated carbon filter.

In an effort to overcome the problems of high temperature and humidity and the discharge of a large quantity of water in a short time, a circulation-condensation type food waste treatment method was proposed. In this method, exhaust gas is condensed by heat exchange to remove moisture therefrom, and then dried exhaust gas is re-supplied into a pulverizing and drying furnace which pulverizes and dries food waste. Water removed from food waste and exhaust gas is drained into a sewer pipe. However, condensate water generated from the exhaust gas contains odorous gas. When condensate water is drained into the sewer pipe, there is the problem of backflow of offensive odors. Furthermore, because discharge of water is not smooth, it takes a longer amount of time to treat food waste. Meanwhile, to solve the problem of odorous condensate water, a method of electrolyze condensate water to deodorize it may be used. However, an electrolyte or electrolytic filter is required to electrolyze condensate water, thus increasing maintenance costs.

### SUMMARY OF THE INVENTION

Accordingly, the present invention has been made keeping in mind the above problems occurring in the prior art, and an object of the present invention is to provide a deodorization module in which a heat exchanger having a stacked structure recovers waste heat from exhaust gas generated from food waste in the drying furnace, and condensate water passes through an electrolytic cell so that offensive odors can be effectively removed from the condensate water using bipolar packed bed electrolysis.

To accomplish the above object, the present invention provides a deodorization module, including a heat exchanger and an electrolytic cell. The heat exchanger is provided outside the drying furnace into which food waste is input. In the heat exchanger, heat is transferred from exhaust gas discharged from the drying furnace to cooling air drawn into the heat exchanger from the outside of the drying furnace. The electrolytic cell electrolyzes condensate water, formed by the heat exchange process of the heat exchanger, using bipolar packed bed electrolysis to remove offensive odors from the condensate water. The heat exchanger has a plurality of flow channels therein so that the exhaust gas discharged from the drying furnace and the cooling air drawn from the outside flow along the flow channels in directions crossing each other.

The electrolytic cell may include a cell housing, an electrode casing installed in the cell housing, and a conductive porous filler charged into the electrode casing. The conductive porous filler may be bipolarized by electricity applied to the interior of the electrode casing and thus deodorize condensate water drawn into the electrode casing.

The electrolytic cell may further include cell electrodes comprising an anode cell and a cathode cell which are disposed in the electrode casing and which face each other. The conductive porous filler may be provided between the cell electrodes.

The heat exchanger may comprise a plurality of unit heat exchange panels, wherein the flow channels may be formed by stacking the unit heat exchange panels one on top of another.

Each of the unit heat exchange panels may include first heat transfer fins extending from a first surface of the unit heat exchange panel, and second heat transfer fins extending from a second surface of the unit heat exchange panel. The first heat transfer fins and the second heat transfer fins may be oriented in directions that cross each other.

Furthermore, when the unit heat exchange panels are coupled to each other, heat transfer fins of each of the unit heat exchange panels may be inserted between heat transfer fins of the adjacent unit heat exchange panel such that the heat transfer fins of one heat exchange panel alternate with the heat transfer fins of the other unit heat exchange panel.

The heat exchanger may include a main body having a plurality of flow channels formed through the main body in a first direction, and a pair of cover plates coupled to respective opposite ends of the main body with respect to the first direction. The flow channels may comprise a plurality of first flow channels through which a first fluid flows in the first direction, the first fluid passing through the cover plates, and a plurality of second flow channels being open through opposite sidewalls of the main body.

The first flow channels and the second flow channels may be alternately formed through the main body.

The first and second flow channels may be formed through the main body in the first direction by extruding.

In addition, heat exchange fins having predetermined shapes may be provided in the first and second flow channels.

The heat exchange fins may be alternately disposed on opposite sides in each of the first and second flow channels.

The electrolytic cell may include a water level sensor attached to the cell housing below the electrode casing, and a discharge pump connected to an outlet formed in a lower end of the cell housing, wherein operation of the discharge pump is controlled depending on a water level detected by the water level sensor.

The water level sensor may comprises a first water level sensor and a second water level sensor disposed below the first water level sensor, wherein the operation of the discharge pump begins when the water level is detected by the first water level sensor, and the operation of the discharge pump is stopped when the water level is detected by the second water level sensor.

The exhaust gas condensed in the heat exchanger may be re-supplied into the drying furnace.

As described above, in a deodorization module according to the present invention, a heat exchanger having a stacked structure recovers waste heat from exhaust gas generated from a drying furnace, and condensate water passes through a bipolar packed bed electrolysis and deodorization device. Therefore, offensive odors can be effectively removed from the condensate water. Furthermore, due to use of the heat exchanger and the electrolysis and deodorization device, power consumption can be markedly reduced compared to that of the conventional technique. Hence, the cost required to maintain the system can be reduced.

Furthermore, the present invention uses the intake and discharge module and the heat exchanger having the stacked structure, so that the time taken to treat food waste can be markedly reduced. In addition, heat efficiency can be enhanced by the operation of re-supplying waste heat into the drying furnace. Thereby, the frequency of operation of the heater in the drying furnace can be reduced. Moreover, because condensate water is treated using bipolar packed bed electrolysis, a separate electrolyte is not required unlike the conventional technique. Also, 95% or more of the odorous gas can be removed. Furthermore, in comparison with the deodorization filter of the conventional technique which must be replaced with a new one once every two to four months, the deodorization module of the present invention can be used semi-permanently, thus reducing the maintenance cost.

### BRIEF DESCRIPTION OF THE DRAWINGS

The above and other objects, features and advantages of the present invention will be more clearly understood from the following detailed description taken in conjunction with the accompanying drawings, in which:
FIG. 1 is a perspective view of a food waste treatment apparatus, according to the present invention;
FIG. 2 is a front view of the apparatus shown from the direction indicated by the arrow A of FIG. 1;
FIG. 3 is a sectional view taken along line B-B of FIG. 1;
FIG. 4 is a sectional view taken along line C-C of FIG. 1;
FIG. 5 is a perspective view showing an embodiment of a heat exchange unit installed in a heat exchanger according to the present invention;
FIG. 6 is a partial exploded perspective view of the heat exchange unit of FIG. 5;
FIG. 7 is a perspective view of a unit heat exchange panel of the heat exchange unit of FIG. 5;
FIG. 8 is a perspective view of another embodiment of a heat exchange unit according to the present invention;
FIG. 9 is an exploded perspective view showing a main body of the heat exchange unit of FIG. 8, the front and rear surfaces from which cover plates are separated;
FIG. 10 is a perspective view of the main body of the heat exchange unit in which second flow channels formed in the main body are exposed to the outside through the sidewalls of the main body by cutting portions of the sidewalls of the main body;
FIG. 11 is a sectional view taken along line F-F of FIG. 10; and
FIG. 12 is a sectional view showing the internal construction of an electrolytic cell according to the present invention.

### DESCRIPTION OF THE PREFERRED EMBODIMENTS

Hereinafter, a deodorization module according to a preferred embodiment of the present invention will be described in detail with reference to the attached drawings.

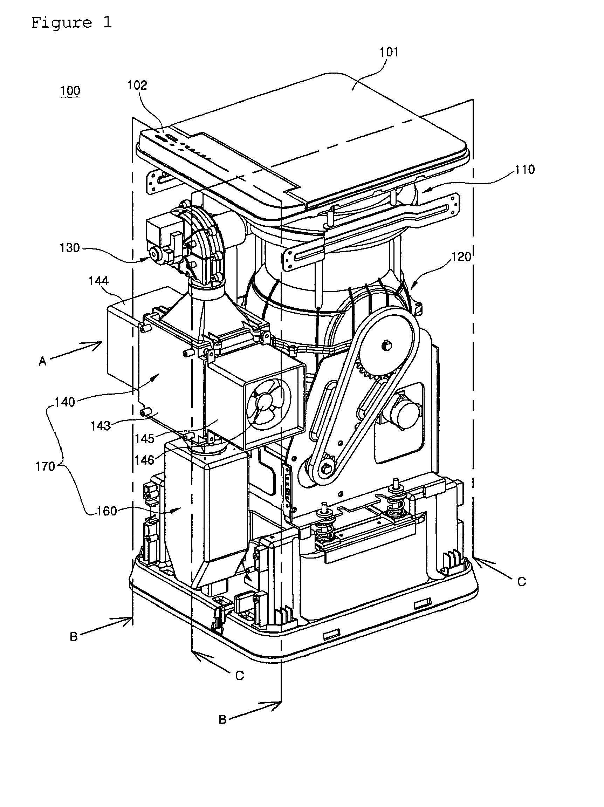

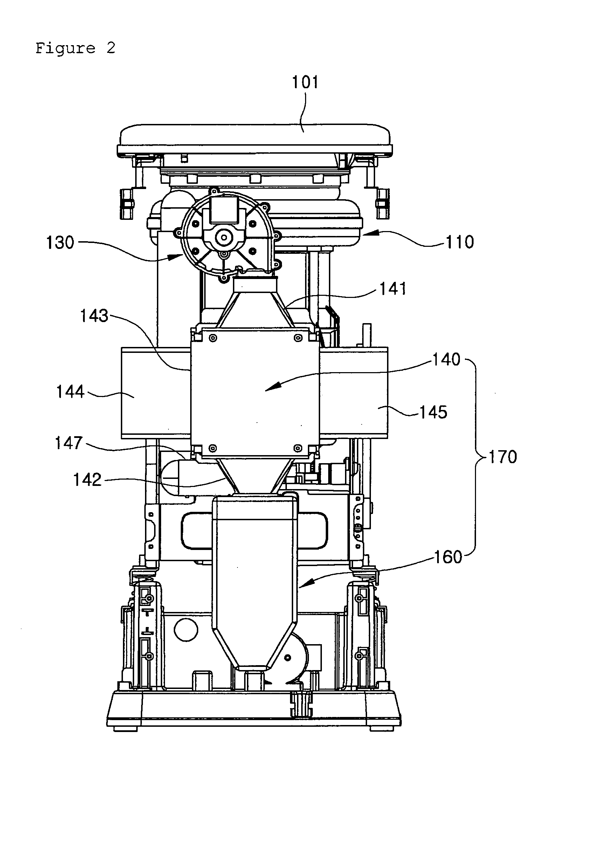

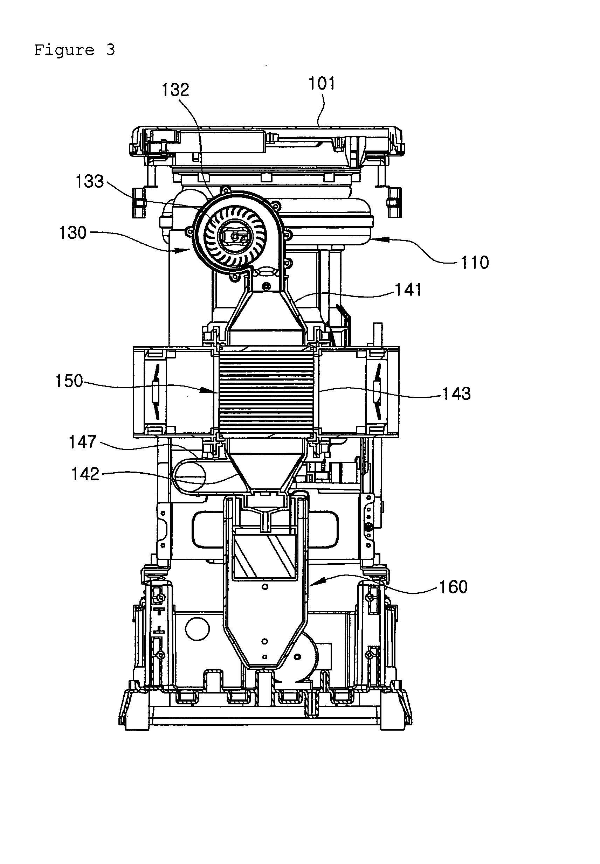

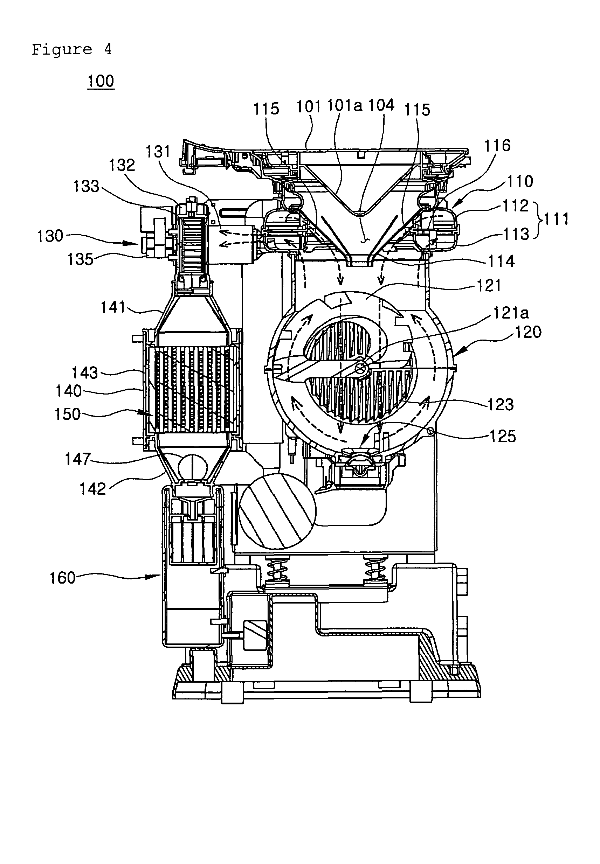

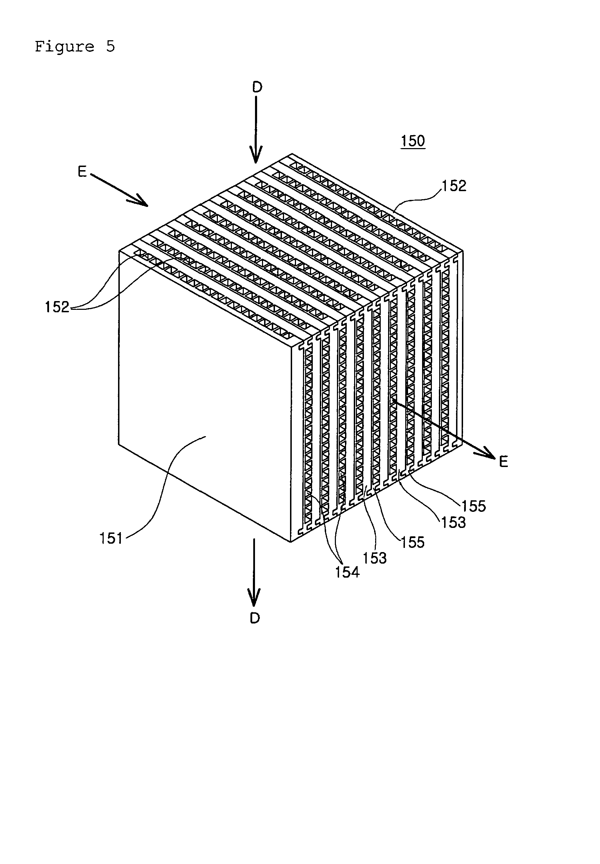

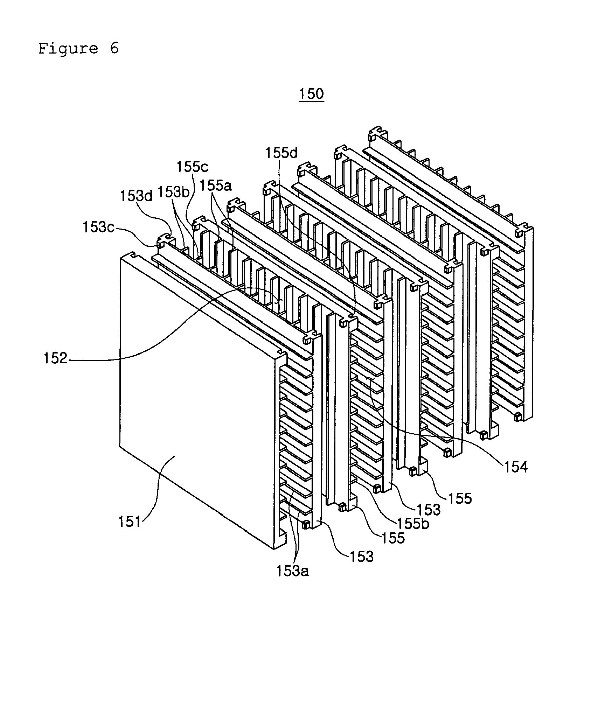

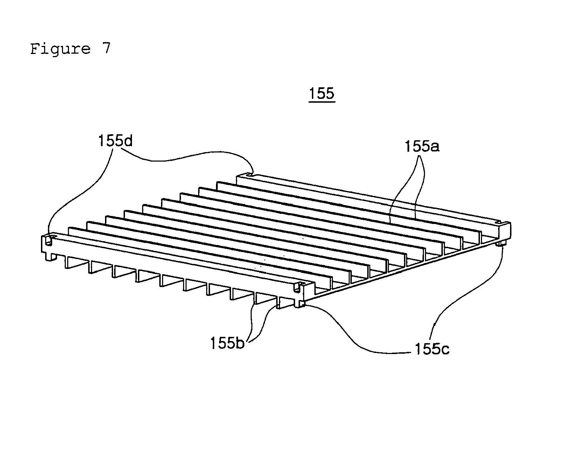

FIG. 1 is a perspective view of a food waste treatment apparatus 100, according to the present invention. FIG. 2 is a front view of the apparatus 100 shown from the direction indicated by the arrow A of FIG. 1. FIG. 3 is a sectional view taken along line B-B of FIG. 1. FIG. 4 is a sectional view taken along line C-C of FIG. 1. FIG. 5 is a perspective view showing an embodiment of a heat exchange unit 150 installed in a heat exchanger 140. FIG. 6 is a partial exploded perspective view of the heat exchange unit 150 of FIG. 5. FIG. 7 is a perspective view of a unit heat exchange panel 155 of the heat exchange unit 150 of FIG. 5.

The general construction of the food waste treatment apparatus 100 according to the embodiment of the present invention will be explained with reference to FIGS. 1 through 4. The food waste treatment apparatus 100 includes a drying furnace 120, an intake and exhaust module 110, the heat exchanger 140, an electrolytic cell 160 and an exhaust unit 130. The drying furnace 120 stirs, pulverizes and heats food waste therein. The intake and exhaust module 110 is coupled to an upper end of the drying furnace 120 to exhaust gas out of the drying furnace 120 or draw outside air into the drying furnace 120. The heat exchanger 140 is installed at a predetermined position adjacent to the drying furnace 120 to transfer the heat of gas exhausted from the drying furnace 120 to cooling air drawn from the outside. The electrolytic cell 160 is disposed under the heat exchanger 140 and electrolyzes condensate water formed during the heat exchange process of the heat exchanger 140 so as to remove offensive odors. The exhaust unit 130 is provided between the intake and exhaust module 110 and the heat exchanger 140 to forcibly transfer gas from the intake and exhaust module 110 into the heat exchanger 140.

Particularly, the heat exchanger 140 and the electrolytic cell 160 serve as a deodorization module 170. The deodorization module 170 cools high temperature and humidity vapor generated from the drying furnace 120 using a stacked heat exchanger structure so that cooling rate is increased and condensate water can be rapidly formed. The condensate water is treated using bipolar packed bed electrolysis, so that offensive odors can be more effectively removed from the condensate water.

Referring to FIG. 4, the drying furnace 120 generally has a hollow spherical or elliptical shape. The drying furnace 120 includes a pulverizing screw 121 which extends from a rotating shaft 121a in a spiral shape, a pulverizing plate 123 which is disposed on one inner surface of the drying furnace 120, and a food waste discharge unit 125 which periodically discharges food waste out of the drying furnace 120. The drying furnace 120 pulverizes and stirs food waste using interaction between the pulverizing screw 121 and pulverizing protrusions (not shown) which are provided on the inner surface of the drying furnace 120. Furthermore, because the drying furnace 120 has the spherical or elliptical shape, treated food waste is smoothly collected in the lower portion of the drying furnace 120, so that removal of the collected food waste from the apparatus can be facilitated.

The intake and exhaust module 110 includes a tube 111 having a hollow ring shape, and a counter-current prevention plate 114 which is provided inside the tube 111 and has a funnel shape which is reduced in diameter from top to bottom. The counter-current prevention plate 114 comprises a plurality of members which are separated from each other. Thus, when food waste is input into an inlet port 104, the counter-current prevention plate 114 expands in diameter. When the drying furnace 120 is being operated, the counter-current prevention plate 114 maintains a diameter-contracted state to prevent odor gas or food waste from flowing backwards.

The tube 111 comprises an intake tube 112 and an exhaust tube 113. Exhaust gas to be reused, containing waste heat, is drawn from the heat exchanger 140 into the intake tube 112. Exhaust gas generated from the drying furnace 120 is exhausted out of the apparatus 100 through the exhaust tube 113. A separation plate 116 is interposed between the intake tube 112 and the exhaust tube 113. The separation plate 116 functions to transfer heat from high temperature and humidity exhaust gas flowing through the exhaust tube 113 to dry exhaust gas which is supplied from the heat exchanger 140 and flows through the intake tube 112. The separation plate 116 is made of an aluminum or stainless steel based material which has a high heat transfer rate. A guide 115 is provided at a position spaced apart from the outer surface of the counter-current prevention plate 114 by a predetermined distance. Dry exhaust gas drawn into the intake tube 112 is guided by the guide 115 into the drying furnace 120 without interference.

Meanwhile, a door 101 is provided above the intake and exhaust module 110. The door 101 includes an input panel 102 through which a user inputs a control signal, and a protrusion 101a which is convex downwards. When vapor containing offensive odors condenses on the door 101, water formed by the condensation is moved downwards along an inclined surface of the protrusion 101a and then dried by heat generated from the drying furnace 120. Thus, contamination on the door 101 attributable to water can be avoided.

The exhaust unit 130 includes an exhaust housing 132, an exhaust fan 133 which is installed in the exhaust housing 132, a motor 135 which provides power to the exhaust fan 133, and a connection pipe 131 which connects the exhaust housing 132 to the exhaust tube 113 of the intake and exhaust module 110.

The heat exchanger 140 includes a heat exchanger housing 143, a heat exchange unit 150 which is installed in the heat exchanger housing 143, an upper inlet port 141 which is coupled to an upper end of the heat exchanger housing 143, and a lower outlet port 142 which is coupled to a lower end of the heat exchanger housing 143. The heat exchanger 140 receives high temperature and humidity exhaust gas from the exhaust unit 130 through the upper inlet port 141, and heat exchange is conducted in the heat exchange unit 150. Thereafter, condensate water formed by the heat exchange moves to the electrolytic cell 160 through the lower outlet port 142. The heat exchanger 140 has a plurality of flow channels therein. The heat exchange is conducted in such a way that high temperature and humidity exhaust gas generated from the dry furnace and cooling air supplied from the outside flow through the flow channels in directions which cross each other.

Hereinafter, one embodiment of the heat exchange unit 150 used in the present invention will be described in detail with reference to FIGS. 5 through 7. The heat exchange unit 150 includes a plurality of unit heat exchange panels 153 and 155 which are stacked one on top of another such that flow channels formed by the unit heat exchange panels 153 and 155 are oriented in directions crossing each other. Thus, high temperature and humidity exhaust gas which is drawn into the heat exchange unit 150 through the upper inlet port 141 and cooling air which is supplied thereinto through an outside air inlet 144 can flow through the heat exchanger 140 in the directions crossing each other so that efficiency of the heat exchange is enhanced. Preferably, the unit heat exchange panels 153 and 155 are stacked one on top of another such that the flow channels thereof are perpendicular to each other.

Each unit heat exchange panel 153, 155 may be formed by die casting. The die casting is a precise casting method in which molten metal is injected into a steel mold which is formed by a precise machining process to have a shape corresponding to a shape of a product to be formed, so that a casting product having the same shape as that of the mold can be formed. The product formed by the die casting has the mechanical characteristic of being precisely measured and requiring no finishing. Furthermore, die casting as such makes mass production possible.

Meanwhile, exhaust gas from which water is removed by condensation while passing through the heat exchange unit 150 is re-supplied into the drying furnace 120 through a return pipe 147 which is disposed between the lower outlet port 142 and the electrolytic cell 160.

The heat exchange unit 150 includes a first outer panel 151, a second outer panel 152 and unit heat exchange panels 153 and 155 which are stacked one on top of another between the first and second outer panels 151 and 152. First heat transfer fins 153a, 155a protrude from a first surface of each unit heat exchange panel 153, 155. The first heat transfer fins 153a, 155a have bar shapes and are spaced apart from each other at regular intervals. Second heat transfer fins 153b, 155b protrude from a second surface of each unit heat exchange panel 153, 155. The second heat transfer fins 153b, 155b also have bar shapes and are spaced apart from each other at regular intervals. Coupling protrusions 153c, 155c are provided on the respective corners of the first surface of each unit heat exchange panel 153, 155. Coupling depressions 153d, 155d corresponding the coupling protrusions 153c, 155c are formed in the respective corners of the second surface of each unit heat exchange panel 153, 155, so that the coupling protrusions 153c and 155c are inserted into the corresponding coupling depressions 153d and 155d of the adjacent unit heat exchange panel 153 or 155. Furthermore, the second heat transfer fins 153b of the first unit heat exchange panels 153 are parallel to the first heat transfer fins 155a of the second unit heat exchange panels 155, and they are configured in such a way that each second heat transfer fin 153b is inserted into the space between the adjacent first heat transfer fins 155a.

In each unit heat exchange panel 153, 155, the first heat transfer fins 153a, 155a are oriented in a direction that crosses the second heat transfer fins 153b, 155b. Preferably, the first heat transfer fins 153a, 155a and the second heat transfer fins 153b, 155b are oriented perpendicular to each other.

For example, referring to FIG. 7, the first and second heat transfer fins 155a and 155b of the second unit heat exchange panel 155 are oriented perpendicular to each other, thus forming flow channels which are perpendicular to each other on opposite sides of the panel 155.

In the heat exchange unit 150 having the above-mentioned stacked structure, high temperature and humidity exhaust gas passes through the exhaust unit 130 in a direction indicated by the arrow D. Cooling air supplied from the outside passes through the exhaust unit 130 in the direction indicated by the arrow E. The arrows D and E indicate the flow directions which are perpendicular to each other. In the heat exchange unit 150, exhaust gas transfers heat energy to cooling air. In the embodiment, flow channels which are formed in the heat exchange unit 150 in the direction of the arrow D are referred to as first flow channels 152. Flow channels which are formed in the heat exchange unit 150 in the direction of the arrow E are referred to as second flow channels 154. The first and second flow channels 152 and 154 are configured in the heat exchange unit 150 such that they alternate with each other to increase efficiency of heat exchange between the exhaust gas and the cooling air.

The first heat transfer fins 153a, 155a (which have high heat conductivity and protrude from each heat exchange panel 153, 155) are placed between the second heat transfer fins 153b, 155b of the adjacent heat exchange panel 153 or 155, so that the distance between the fins is reduced. Thereby, efficiency of heat exchange can be increased. In the conventional art, because the area of the high temperature fluid flowing part is less than that of the cooling fin, the resistance of fluid is comparatively large. In addition, a contact area of vapor for condensation is comparatively small, so that the efficiency of condensation is low. However, in the present invention, a heat exchange area between the cooling side and the high temperature side is increased five fold or greater compared to the conventional technique. Furthermore, the area of the flow channel is comparatively large, so that flow resistance and a fluid rate are reduced. As a result, the efficiency of condensation is increased.

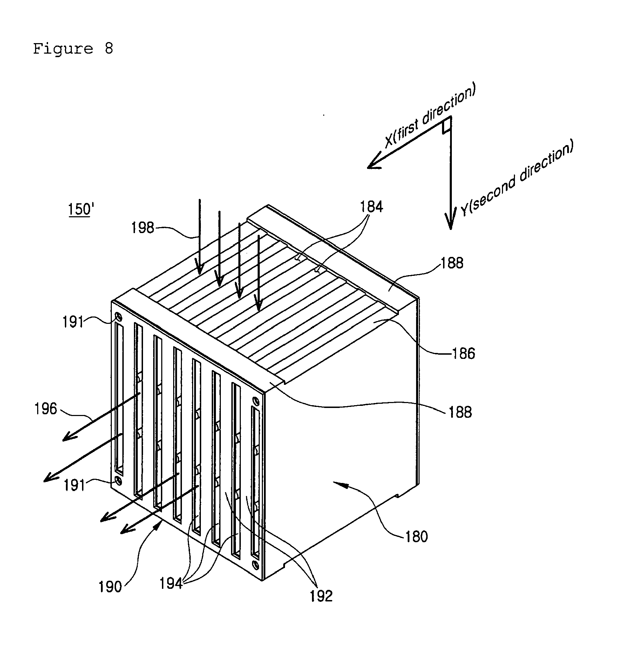

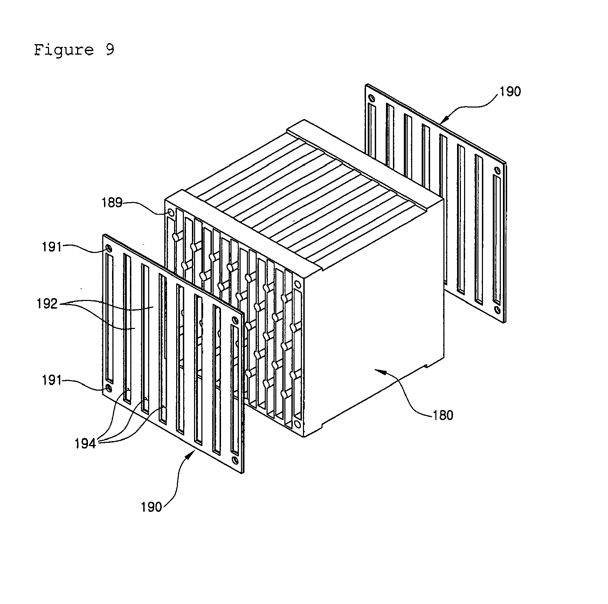

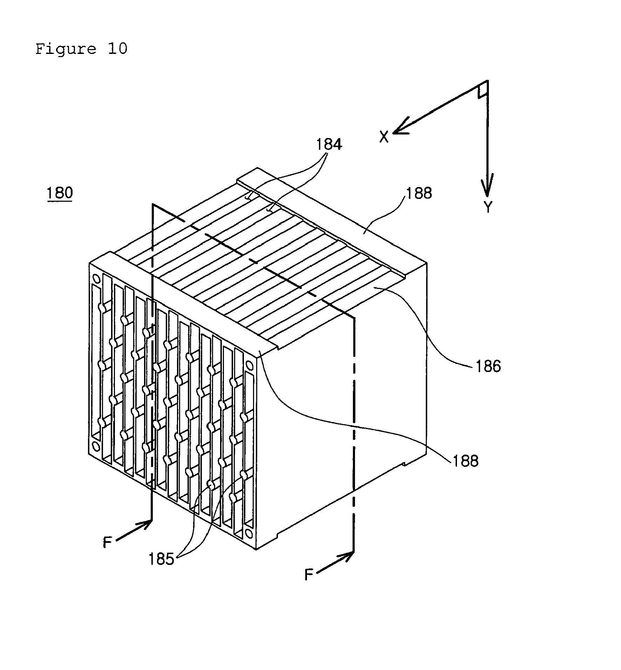

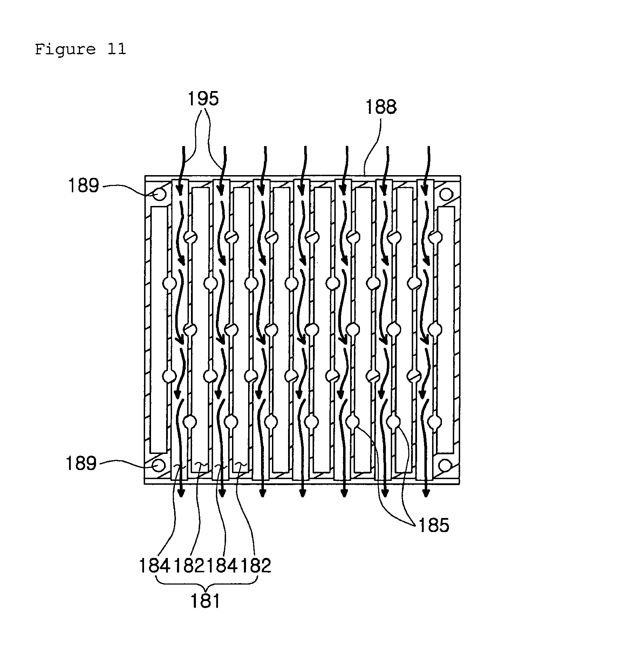

FIG. 8 is a perspective view of another embodiment of a heat exchange unit 150' according to the present invention. FIG. 9 is an exploded perspective view showing a main body 180 of the heat exchange unit 150' of FIG. 8, the front and rear surfaces from which cover plates 190 are separated. FIG. 10 is a perspective view of the main body 180 of the heat exchange unit 150' in which second flow channels formed in the main body 180 are exposed to the outside through the sidewalls of the main body 180 by cutting portions of the sidewalls of the main body 180. FIG. 11 is a sectional view taken along line F-F of FIG. 10.

Hereinafter, the embodiment of the heat exchange unit 150' according to the present invention will be described in detail with reference to FIGS. 8 through 11.

The heat exchange unit 150' includes the main body 180 which has a plurality of flow channels 181, and the cover plates 190 which are respectively coupled to the front and rear surface of the main body 180. Fastening depressions 189 are formed in the respective corners of the front and rear surfaces of the main body 180. Fastening holes 191 are formed through the respective corners of the cover plates 190 at positions corresponding to the fastening depressions 189. The cover plates 190 are coupled to the main body 180 by tightening separate fastening members (not shown) into the fastening holes 191 and the fastening depressions 189.

The main body 180 is formed by extruding into a single body, so that the main body 180 has a similar cross-section over its entire length. The extruding process can reduce the time it takes to produce the main body 180.

The flow channels 181 comprise first flow channels 182 which function as passages for first fluid passing through the cover plates 190, and second flow channels 184 which are formed through the opposite sidewalls of the main body 180. As shown in FIG. 8, the direction in which the first fluid moves through the front and rear surfaces of the main body 180 is designated by a first direction 196. The direction in which second fluid moves through the upper and lower sidewalls of the main body 180 is designated by a second direction 198. For example, in rectangular coordinates, the X-axis denotes the first direction and the Y-axis denotes the second direction. In the embodiment, the first and second directions 196 and 198 have been illustrated as being perpendicular to each other for the sake of description, though they need not be oriented perpendicular to each other.

The first fluid is drawn into the rear surface of the main body 180 in the first direction 196, passes through the first flow channels 182, and then moves out of the main body 180 through the front surface of the main body 180. The cover plates 190 do not interfere with the flow of the first fluid.

The second fluid is drawn into the upper sidewall of the main body 180 in the second direction 198, passes through the second flow channels 184, and then moves out of the main body 180 through the lower sidewall thereof. The second flow channels 184 are open to the outside through the upper and lower sidewalls of the main body 180 by cutting off portions of the upper and lower sidewalls of the main body 180. Stepped portions are formed on the upper and lower sidewalls of the main body 180 by the cutting process. The stepped portion is formed by a difference in depth between a base surface 188 which is not involved in the cutting process and a processed surface 186 which is involved in the cutting process. The second flow channels 184 are open to the outside through the upper and lower sidewalls of the main body 180 because of the stepped portions.

The cover plates 190 prevent the second fluid from being discharged out of the main body 180 through the front or rear surface of the main body 180. In other words, the cover plates 190 close openings of the second flow channels 184 which are formed on the front and rear surfaces of the main body 180. The first fluid and the second fluid respectively denote exhaust gas discharged from the exhaust unit 130 and cooling air drawn from the outside.

Slots 194 are formed through each cover plate 190 so that the first fluid can be drawn into and discharged out of the main body 180 through the slots 194 in the first direction 196. The slots 194 may be formed through the cover plate 190 by piercing a metal plate using a press machine. Furthermore, each cover plate 190 has blocking portions 192 which are provided between the slots 194. The blocking portions 192 prevent the second fluid from leaking out of the front and rear surfaces of the main body 180. In addition, a sealing member (not shown) is attached to the inner surface of the cover plate 190, so that when the cover plate 190 is tightly coupled to the main body 180, the sealing member can prevent fluid from leaking between the cover plate 190 and the main body 180.

As shown in FIG. 11, the first flow channels 182 and the second flow channels 184 which are formed in the main body 180 alternate with each other. In other words, the first and second flow channels 182 and 184 are configured such that two second flow channels 184 are respectively formed on opposite sides of each first flow channel 182. Thus, heat exchange can be smoothly conducted between the first fluid which flows through the first flow channels 182 and the second fluid which flows through the second flow channels 184. In the embodiment, heat exchange fins 185 having predetermined shapes are provided between the flow channels 182 and 184 to further enhance heat exchange efficiency between the first and second fluids.

Referring to FIG. 11, the second fluid which is drawn into the second flow channel 184 in the second direction 198 moves in a streamlined form due to the heat exchange fins 185. In addition, the second fluid comes into close contact with the surfaces of the second flow channels 184 when it is moving through the second flow channels 184. Thereby, heat transfer efficiency between the flow channels 181 can be enhanced. Preferably, to ensure the streamlined flow pattern of the second fluid, the heat exchange fins 185 are arranged along the second direction 198 such that they are alternately disposed on opposite sides of each second flow channel 184.

Meanwhile, the flow channels 181 which are formed through the main body 180 by the extruding process have different lengths with respect to the second direction 196; in detail, the second flow channels 184 are longer than the first flow channels 182 with respect to the second direction 196 so that upper and lower ends of the second flow channels 184 are closer to the upper and lower sidewalls of the main body 180 than are the first flow channels 182. Thus, when the process of cutting off the portions of the upper and lower sidewalls of the main body 180 is conducted, only the upper and lower ends of the second flow channels 184 are open to the outside without the upper or lower ends of the first flow channels 182 being exposed.

In this state, second fluid which is drawn into the main body 180 through the upper sidewall thereof passes through the second flow channels 184 before moving out of the lower sidewalls of the main body 180. When the second fluid is passing through the second flow channels 184, it flows in a streamlined zigzag form due to the protruding shape of the heat exchange fins 185 (refer to reference numeral 195 of FIG. 11).

As described above, in the heat exchange unit 150 or 150' according to the present invention, the flow channels 152 and 154 or 182 and 184 cross each other or are perpendicular to each other, so that heat exchange efficiency between the exhaust gas and the cooling air can be enhanced.

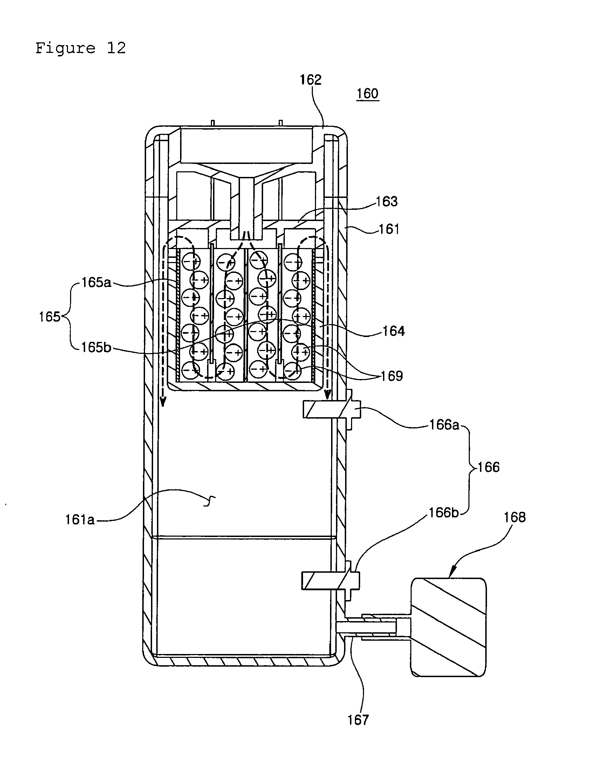

FIG. 12 is a sectional view showing the internal construction of the electrolytic cell 160. Hereinafter, the construction of the electrolytic cell 160 will be explained with reference to FIG. 12. The electrolytic cell 160 includes a hollow cell housing 161, a housing cover 162 which is coupled to an upper end of the cell housing 161, and an electrode casing 164 which is installed in the cell housing 161. The electrolytic cell 160 further includes a cell cover 163 which covers an upper end of the electrode casing 164, a water level sensor 166 which is attached to the cell housing 161 below the electrode casing 164, and a discharge pump 168 which is connected to an outlet 167 formed in a lower end of the cell housing 161.

In the electrode casing 164, cell electrodes 165 are arranged such that they face each other. The cell electrodes 165 comprise an anode cell 165a and a cathode cell 165b. A space between the anode cell 165a and the cathode cell 165b is filled with conductive porous fillers 169 or a mixture containing conductive porous fillers 169.

Condensate water cooled in the heat exchanger 140 is drawn into the electrode casing 164 and passes through an internal path which is formed in a zigzag shape to increase a contract area and the time involved in electrolysis. In this structure, each particle of conductive porous fillers 169 which are charged in the electrode casing 164 functions as a bipolar electrode. Therefore, the overall area of the entire electrode is maximized, and the electrolysis reaction occurs in the entirety of the electrode casing 164, thus minimizing the presence of an unreacted odorous substance.

In the electrolysis method using the bipolar packed bed electrolytic cell according to the present invention, electricity is applied only to the cell electrodes 165 having opposite polarities rather than being directly applied to the conductive porous fillers 169, so that the conductive porous fillers 169 are changed into bipolar electrodes, thus increasing the active electrode area, thereby enhancing the efficiency of electrolysis.

Furthermore, fine ionic material is eluted from the conductive porous fillers 169 during the electrolysis. The ionic material increases the conductivity of condensate water and maintains it constant, thus enhancing the efficiency of the electrolysis, and stabilizing control of the electrolysis. Moreover, a conductive material, such as activated carbon, alumina or stainless steel powder, etc., is used as the conductive porous fillers 169. In addition, the conductive porous fillers 169 exhibit an adsorptive effect using pores formed therein, thus increasing deodorization efficiency.

The operation of the food waste treatment apparatus 100 of the present invention will be explained with reference to FIGS. 1 through 12.

Food waste is input into the drying furnace 120. The pulverizing screw 121 stirs and pulverizes the food waste and, simultaneously, a heater (not shown) provided in the drying furnace 120 dries the food waste. Exhaust gas containing offensive odors generated from the drying furnace 120 is transferred to the heat exchanger 140 by the operation of the exhaust unit 130. In the heat exchanger 140, high temperature and humidity exhaust gas which is drawn into the heat exchanger 140 through the upper inlet port 141 transfers heat to cooling air which is drawn into the heat exchanger 140 through the outside air inlet 144. The exhaust gas that is dried in the heat exchanger 140 is re-supplied into the drying furnace 120 via the return pipe 147 and the intake tube 112 of the heat exchanger 140. Water formed by condensation of vapor in the heat exchanger 140 is moved into the electrolytic cell 160.

The exhaust gas which is reduced in temperature by primary heat exchange and cooling in the heat exchanger 140 is slightly increased in temperature by a secondary heat exchange process wherein the exhaust gas receives heat, in the intake tube 112 of the intake and exhaust module 110, from high temperature exhaust gas which flows through the exhaust tube 113. As such, heat efficiency is enhanced by a multi-step heat exchange process, so that the efficiency of the condensation process and the heat recovery process can be enhanced compared to the conventional technique.

Condensed odorous water which is drawn into the electrolytic cell 160 passes through the conductive porous fillers 169 provided between the cell electrodes 165 including the anode cell 165a and the cathode cell 165b. In this way, odorous gas ingredients are removed from the condensed odorous water by the electrolysis and adsorption operation of the conductive porous fillers 169. Here, an electrolytic reaction occurs in the entire space inside the electrode casing 164 containing the conductive porous fillers 169 rather than partially occurring on the surface of the cell electrodes 165. Meanwhile, although the electrolytic reaction is insignificant when only condensate water is used, because the gap between the conductive porous fillers 169 is very small, electricity is easily transmitted despite low voltage and current. Hence, electrolysis can be conducted without adding a separate electrolyte.

Condensate water treated through the electrode casing 164 is stored in a buffer space 161a of the cell housing 161. When a first water level sensor 166a detects that condensate water charged into the buffer space 161a has reached a predetermined water level, the discharge pump 168 is operated, so that the condensate water is discharged out of the cell housing 161 through an outlet 167. When the level of the condensate water has reached a height corresponding to a second water level sensor 166b, the discharge pump 168 is stopped. Due to this structure, air can be prevented from being drawn into the discharge pump 168, so that the reliability of operation can be ensured.

As described above, in the present invention, the heat exchanger 140 having the stacked structure recovers waste heat from exhaust gas generated from the drying furnace 120, and condensate water passes through the electrolytic cell 160 which functions as a bipolar packed bed electrolysis and deodorization device. Therefore, offensive odors can be effectively removed from the condensate water. Furthermore, due to the use of the deodorization module 170 including the heat exchanger 140 and the electrolytic cell 160, power consumption can be markedly reduced. Hence, the cost required to maintain the system can be reduced.

Although the preferred embodiment of the present invention has been disclosed for illustrative purposes, the present invention is not limited to the embodiment. Furthermore, those skilled in the art will appreciate that various modifications, additions and substitutions are possible, without departing from the scope and spirit of the invention as disclosed in the accompanying claims. These modifications, additions and substitutions must be regarded as falling within the bounds of the claims.

## Claims

1. A deodorization module, comprising:
a heat exchanger conducting heat exchange between an exhaust gas discharged from a drying furnace and cooling air drawn into the heat exchanger from an outside of the drying furnace; and
an electrolytic cell electrolyzing condensate water, formed by the heat exchange process of the heat exchanger, using bipolar packed bed electrolysis to remove offensive odors from the condensate water, wherein
the heat exchanger has a plurality of flow channels therein so that the exhaust gas discharged from the drying furnace and the cooling air drawn from the outside flow along the flow channels in directions that cross each other.

2. The deodorization module as set forth in claim 1, wherein the electrolytic cell comprises:
a cell housing;
an electrode casing installed in the cell housing; and
a conductive porous filler charged into the electrode casing, the conductive porous filler being bipolarized by electricity applied to an interior of the electrode casing.

3. The deodorization module as set forth in claim 2, wherein the electrolytic cell further comprises:
cell electrodes comprising an anode cell and a cathode cell which are disposed in the electrode casing and face each other, with the conductive porous filler provided between the cell electrodes.

4. The deodorization module as set forth in claim 1, wherein the heat exchanger comprises a plurality of unit heat exchange panels, wherein the flow channels are formed by stacking the unit heat exchange panels one on top of another.

5. The deodorization module as set forth in claim 4, wherein each of the unit heat exchange panels comprises first heat transfer fins extending from a first surface of the unit heat exchange panel, and second heat transfer fins extending from a second surface of the unit heat exchange panel, wherein the first heat transfer fins and the second heat transfer fins are oriented in directions that cross each other.

6. The deodorization module as set forth in claim 4, wherein when the unit heat exchange panels are coupled to each other, heat transfer fins of each of the unit heat exchange panels are inserted between heat transfer fins of the adjacent unit heat exchange panel such that the heat transfer fins of one heat exchange panel alternate with the heat transfer fins of the other unit heat exchange panel.

7. The deodorization module as set forth in claim 1, wherein the heat exchanger comprises:
a main body having a plurality of flow channels formed through the main body in a first direction; and
a pair of cover plates coupled to respective opposite ends of the main body with respect to the first direction, wherein
the flow channels comprise a plurality of first flow channels through which a first fluid flows in the first direction, the first fluid passing through the cover plates, and a plurality of second flow channels being open through opposite sidewalls of the main body.

8. The deodorization module as set forth in claim 7, wherein the first flow channels and the second flow channels are alternately formed through the main body.

9. The deodorization module as set forth in claim 7, wherein the first and second flow channels are formed through the main body in the first direction by extruding.

10. The deodorization module as set forth in claim 7, wherein heat exchange fins having predetermined shapes are provided in the first and second flow channels.

11. The deodorization module as set forth in claim 10, wherein the heat exchange fins are alternately disposed on opposite sides in each of the first and second flow channels.

12. The deodorization module as set forth in claim 2, wherein the electrolytic cell comprises a water level sensor attached to the cell housing below the electrode casing, and a discharge pump connected to an outlet formed in a lower end of the cell housing, wherein operation of the discharge pump is controlled depending on a water level detected by the water level sensor.

13. The deodorization module as set forth in claim 12, wherein the water level sensor comprises a first water level sensor and a second water level sensor disposed below the first water level sensor, wherein the operation of the discharge pump begins when the water level is detected by the first water level sensor, and the operation of the discharge pump is stopped when the water level is detected by the second water level sensor.

14. The deodorization module as set forth in claim 1, wherein the exhaust gas condensed in the heat exchanger is re-supplied into the drying furnace.
